# EUROPÄISCHE PATENTSCHRIFT

(11) **EP 1 250 318 B1**
(45) Veröffentlichungstag und Bekanntmachung des Hinweises auf die Patenterteilung: **30.11.2005**
(21) Anmeldenummer: 01942357.3
(22) Anmeldetag: 11.01.2001
(51) Int. Cl.: C07C 269/00, C07C 271/12, C07C 255/54

(54) **VERFAHREN ZUR HERSTELLUNG VON ARYL-IMINOMETHYL-CARBAMINSÄUREESTERN**
METHOD FOR PRODUCING ARYL-IMINOMETHYL-CARBAMIC ACID ESTERS
PROCEDE DE PRODUCTION D'ESTERS D'ACIDE ARYL-IMINOMETHYL-CARBAMIQUE

(30) Priorität: 12.01.2000 DE 10000907
(43) Veröffentlichungstag der Anmeldung: 23.10.2002
(73) Patentinhaber: Boehringer Ingelheim Pharma GmbH & Co.KG, 55218 Ingelheim am Rhein (DE)
(72) Erfinder: BRANDENBURG, Jörg, 65199 Wiesbaden (DE); SOYKA, Rainer, 88400 Biberach (DE); SCHMID, Rolf, 88487 Baltringen (DE); ANDERSKEWITZ, Ralf, 88471 Laupheim (DE); BAUER, Rolf, 55218 Ingelheim am Rhein (DE); HAMM, Rainer, 55218 Ingelheim am Rhein (DE); KRÖBER, Jutta, 55411 Bingen (DE)
(86) Internationale Anmeldenummer: PCT/EP2001/000262
(87) Internationale Veröffentlichungsnummer: WO 2001/051457

(56) Entgegenhaltungen:
- EP-A- 0 518 818
- WO-A-96/02497

## Beschreibung

Die Erfindung betrifft ein großtechnisch anwendbares Verfahren zur Herstellung von Verbindungen der Formel (I) 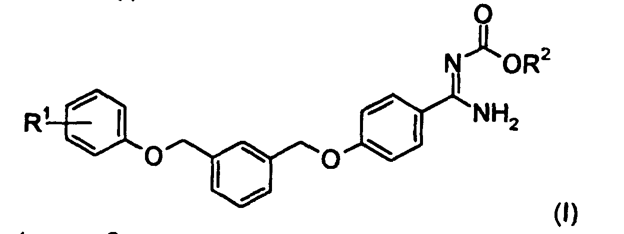 worin die Reste R¹ und R² die in der Beschreibung und in den Ansprüchen genannten Bedeutungen haben können.

### Hinterarund der Erfindung

Aus der Internationalen Patentanmeldung WO 96/02497 sind Benzamidine sowie Aryl-iminomethylcarbaminsäureester bekannt, die eine hohe Wirksamkeit als Arzneistoffe mit LTB₄-antagonistischer Wirkung aufweisen. Von besonderer Bedeutung sind dabei Verbindungen der Formel (I).

Es ist Aufgabe der vorliegenden Erfindung, ein Verfahren bereitzustellen, welches die großtechnische Synthese von Verbindungen der Formel (I) in hohen Ausbeuten und bei hoher Reinheit der Endprodukte erlaubt.

### Detaillierte Beschreibung der Erfindung

Zur Lösung der vorstehend genannten Aufgabe wird erfindungsgemäß ein Verfahren zur Herstellung von Verbindungen der Formel (I) vorgeschlagen 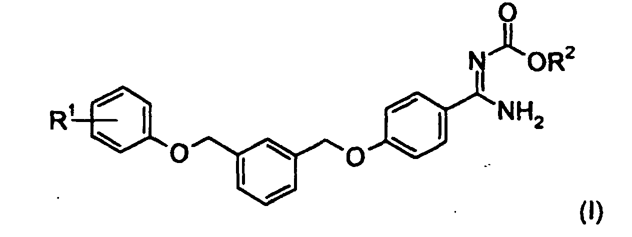 worin
- R¹: ein Rest ausgewählt aus der Gruppe bestehend aus Methyl, Ethyl, Propyl, Cyclopentyl, Cyclohexyl, Phenyl, Benzyl und -C(Me₂)Phenyl bedeutet, der jeweils ein zwei oder dreifach durch Hydroxy substituiert sein kann;
- R²: ein Rest ausgewählt aus der Gruppe bestehend aus Methyl, Ethyl, Propyl und Benzyl bedeutet,
dadurch gekennzeichnet, daß eine Verbindung der Formel (II) 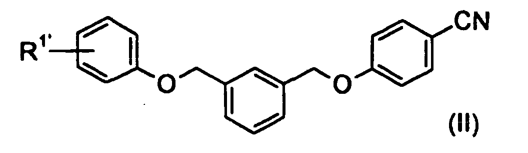 worin
- R^{1'}: ein Rest ausgewählt aus der Gruppe bestehend aus Methyl, Ethyl, Propyl, Cyclopentyl, Cyclohexyl, Phenyl, Benzyl und -C(Me₂)Phenyl bedeutet, der jeweils ein zwei oder dreifach durch eine Gruppe -O-PG substituiert sein kann,
wobei die Gruppe -O-PG für eine geschützte Hydroxylfunktion ausgewählt aus der Gruppe bestehend aus Methoxymethyloxy, 2-Methoxyethoxymethyloxy, 1-Ethoxyethyloxy, 2-Tetrahydropyranyloxy , 1-Butoxyethyloxy, tert.-Butyloxy, Benzyloxy und 4-Methoxybenzyloxy steht,
zunächst in einem etherischen oder aromatischen Lösemittel mit einem Alkalimetall-Hexaalkyldisilazan umgesetzt und anschließend mit einer Verbindung der Formel (III)

R²-O-COX' (III)

worin
- R²: die vorstehend genannte Bedeutung aufweist und
- X': Chlor, Brom oder -O-R² bedeutet, behandelt wird,
nach Aufarbeitung mittels einer Säure der Formel HY eine Verbindung der Formel (IV) 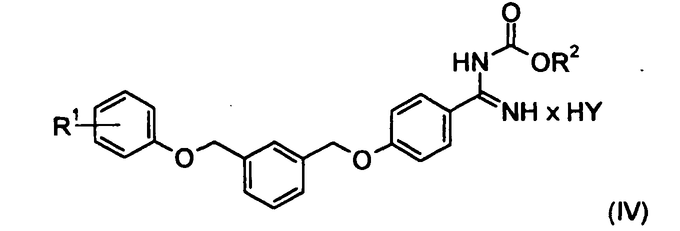 worin die Reste R¹ und R² die vorstehend genannte Bedeutung aufweisen, und Y einen beliebigen Säurerest bedeutet,
isoliert wird und aus dieser die Verbindung der Formel (I) freigesetzt wird.

Die Verbindungen der Formeln (I) und (IV) umfassen auch die entsprechenden Tautomeren der Formeln (I-T) und (IV-T):

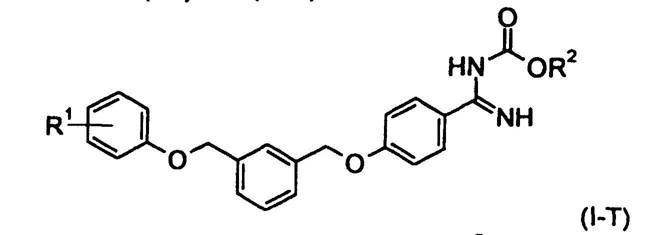

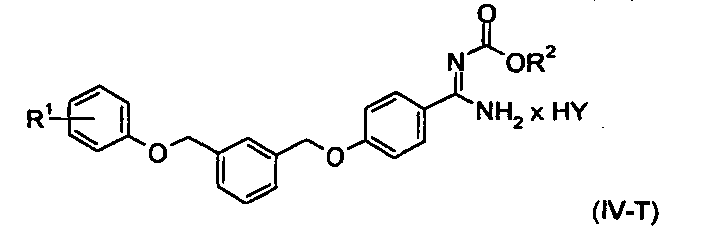

Der Begriff "Alkalimetall-Hexaalkyldisilazan" wie er vor und nachstehend für die Bezeichung des Reagenzes verwendet wird" das mit der Verbindung der Formel (II) umgesetzt wird, bezeichnet in der Regel eine Verbindung der Formel (VIII), 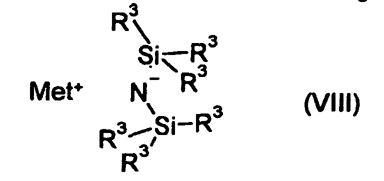 worin
Met für ein Alkalimetall, vorzugsweise Lithium, Natrium oder Kalium, insbesondere Lithium steht, und
R³ jeweils unabhängig für eine C₁-C₄ Alkylgruppe, vorzugsweise Methyl oder Ethyl, insbesondere Methyl steht.

Ganz besonders bevorzugt sind Lithium-hexamethyldisilazan, Natriumhexamethyldisilazan und Kalium-hexamethyldisilazan, insbesondere Lithium-hexamethyldisilazan.

Der Ausdruck "anschließende Umsetzung" mit einer Verbindung der Formel (III) umfasst sowohl Vorgehensweisen, bei denen das Produkt der Umsetzung der Verbindung der Formel (II) mit dem Alkalimetall-Hexamethyldisilazan direkt, ohne weitere Zwischenumsetzung, mit der Verbindung der Formel (III) zur Reaktion gebracht wird, als auch solche wobei Vorgehensweisen, wobei aus dem gebildeten Produkt die freie Amidinbase zwischenzeitlich freigesetzt wird. Vorzugsweise wird das Produkt der Umsetzung der Verbindung der Formel (II) mit dem Alkalimetall-Hexamethyldisilazan direkt, insbesondere in einer "Ein-Topf-Synthese" mit der Verbindung der Formel (III) umgesetzt.

Bevorzugt ist ein Verfahren zur Herstellung von Verbindungen der Formel (I), worin
- R¹: ein Rest ausgewählt aus der Gruppe bestehend aus Phenyl, Benzyl und -C(Me₂)Phenyl bedeutet, der jeweils ein oder zweifach, bevorzugt einfach durch Hydroxy substituiert sein kann;
- R²: ein Rest ausgewählt aus der Gruppe bestehend aus Ethyl, Propyl und Benzyl bedeutet,
dadurch gekennzeichnet, daß eine Verbindung der Formel (II), worin
- R^{1'}: ein Rest ausgewählt aus der Gruppe bestehend aus Phenyl, Benzyl und -C(Me₂)Phenyl bedeutet, der jeweils ein oder zweifach, bevorzugt einfach durch eine Gruppe -O-PG substituiert sein kann,
wobei die Gruppe -O-PG für eine geschützte Hydroxylfunktion ausgewählt aus der Gruppe bestehend aus Methoxymethyloxy, 2-Methoxyethoxymethyloxy, 1-Ethoxyethyloxy, 2-Tetrahydropyranyloxy , 1-Butoxyethyloxy, tert.-Butyloxy, Benzyloxy und 4-Methoxybenzyloxy, bevorzugt für
2-Tetrahydropyranyloxy, steht,
zunächst in einem etherischen oder aromatischen Lösemittel mit einem Alkalimetall-Hexaalkyldisilazan umgesetzt und anschließend mit einer Verbindung der Formel (III)

R²-O-COX' (III)

worin
- R²: die vorstehend genannte Bedeutung aufweist und
- X': Chlor, Brom oder -O-R² bedeutet, behandelt wird,
nach Aufarbeitung mittels wässriger Salzsäure eine Verbindung der Formel (IVA), 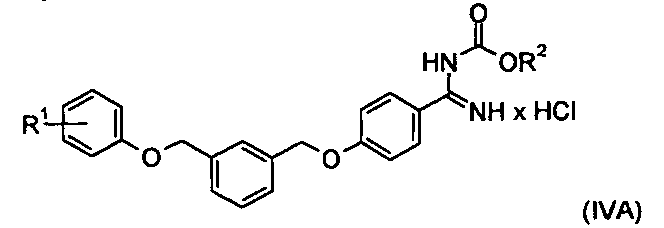 worin die Reste R¹ und R² die vorstehend genannte Bedeutung aufweisen, isoliert wird, und daraus die Verbindung der Formel (I) freigesetzt wird.

Besonders bevorzugt ist ein Verfahren zu Herstellung von Verbindungen der Formel (I), worin
- R¹: -C(Me₂)Phenyl bedeutet, das gegebenenfalls einfach durch Hydroxy substituiert sein kann und
- R²: Ethyl bedeutet,
dadurch gekennzeichnet, daß eine Verbindung der allgemeinen Formel (II)
worin
- R^{1'}: -C(Me₂)Phenyl bedeutet, das gegebenenfalls einfach durch eine Gruppe -O-PG substituiert sein kann, wobei die Gruppe -O-PG für eine geschützte Hydroxylfunktion ausgewählt aus der Gruppe bestehend aus Methoxymethyloxy, 2-Tetrahydropyranyloxy , 1-Butoxyethyloxy, tert.-Butyloxy, Benzyloxy und 4-Methoxybenzyloxy, bevorzugt für 2-Tetrahydropyranyloxy, steht,
zunächst in einem etherischen oder aromatischen Lösemittel mit einem Alkalimetall-Hexaalkyldisilazan umgesetzt und anschließend mit einer Verbindung der Formel (III)

R²-O-COX' (III)

worin
- R²: die vorstehend genannte Bedeutung aufweist und
- X': Chlor, Brom oder -O-R², bevorzugt Chlor, bedeutet, behandelt wird,
nach Aufarbeitung mittels wässriger Salzsäure eine Verbindung der Formel (IVA)
worin die Reste R¹ und R² die vorstehend genannte Bedeutung aufweisen, isoliert wird und aus dieser die Verbindung der Formel (I) freigesetzt wird.

In einer besonders bevorzugten Ausführungsform des erfindungsgemäßen Verfahrens wird dabei die Verbindung der Formel (II) in einem Verfahren hergestellt, welches folgende Schritte umfasst:
(a) Umsetzung von 3-Halogenmethylbenzoesäure-C₁-C₄-alkylestern mit 4-Hydroxybenzonitril im Sinne einer Wilkinson-Ethersynthese;
(b) reduktive Überführung der erhaltenen 3-(4-Cyano-phenoxy)-benzoesäurealkylestern der Formel (VII) 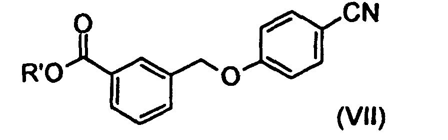 worin R' C₁-C₄-Alkyl bedeutet, in eine Verbindung der Formel (V), 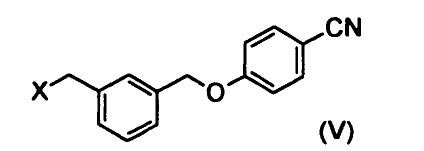 worin X für Hydroxy steht;
(c) gegebenenfalls Behandlung der Verbindung der Formel (V), worin X für Hydroxy steht mit einem Halogenierungsreagenz oder einem Sulfonsäurechlorid; .
(d) Umsetzung der Verbindung der Formel (V), worin X Hydroxy, Chlor, Brom, Mesylat, Triflat oder Tosylat bedeutet, mit einem Phenol Derivat der Formel (VI) 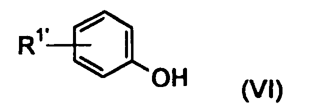
worin R^{1'} die in den Ansprüchen 1 bis 4 genannten Bedeutungen aufweist; gegebenenfalls in Form der entsprechenden Natrium- oder KaliumPhenolate, unter basischen Reaktionsbedingungen vorzugsweise in einem polaren organischen Lösemittel.

Eine zentrale Bedeutung für das erfindungsgemäße Herstellverfahren der Verbindungen der Formel (I) haben die Hydrochloride der Formel (IVA). Sie fallen direkt als gut kristallisierende Salze in hoher Ausbeute an, von denen Nebenprodukte und/oder Verunreinigungen leicht durch Kristallisation abgetrennt werden können. Entspechend zielt ein Aspekt der vorliegenden Erfindungen auf Zwischenprodukte der Formel (IVA) 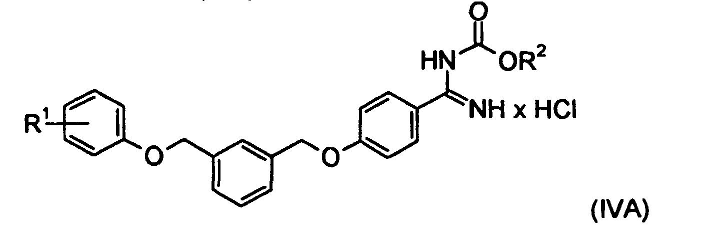 worin die Reste R¹ und R² die vorstehend genannten Bedeutungen haben können. Von den Verbindungen der Formel (IVA) ist die Verbindung
{[4-(3-{4-[1-(4-Hydroxy-phenyl)-1-methyl-ethyl]-phenoxymethyl}-benzyloxy)-phenyl]-imino-methyl}-carbaminsäureethylester-Hydrochlorid besonders bevorzugt.

Die Verbindungen der Formel (II) 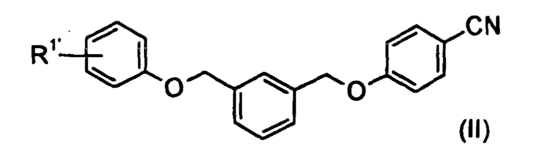 worin R^{1'} die vorstehend genannten Bedeutungen aufweisen kann werden erfindungsgemäß durch Umsetzung einer Verbindung der Formel (V) 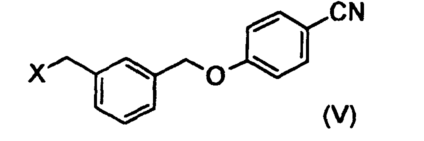 worin X Hydroxy, Chlor, Brom, Mesylat, Triflat, Benzolsulfonat oder Tosylat bedeutet, mit einer Verbindung der Formel (VI) 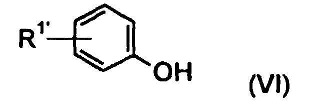 worin R^{1'} die vorstehend genannten Bedeutungen haben kann und wobei die Verbindung der Formel (VI) gegebenenfalls auch in Form ihrer Natrium- und KaliumPhenolate eingesetzt werden kann, unter basischen Reaktionsbedingungen in einem polaren organischen Lösemittel erhalten.

Bevorzugt ist die Darstellung der Verbindungen der Formel (II), worin R^{1'} die vorstehend genannten Bedeutungen aufweisen kann, durch Umsetzung einer Verbindung der Formel (V), worin X Hydroxy, Chlor oder Mesylat, besonders bevorzugt Hydroxy oder Chlor, höchst bevorzugt Chlor bedeutet, mit einer Verbindung der Formel (VI), worin R^{1'} die vorstehend genannten Bedeutungen haben kann und wobei die Verbindung der Formel (VI) in Form ihrer Alkalimetall-Phenolate, bevorzugt in Form ihrer Natriumphenolate eingesetzt wird.

Bei der erfindungsgemäßen Synthese der Verbindungen der Formel (I) kommt den Zwischenprodukten der Formel (V) eine zentrale Bedeutung zu. Dementsprechend zielt ein weiterer Aspekt der vorliegenden Erfindung auf die Verbindungen der Formel (V) 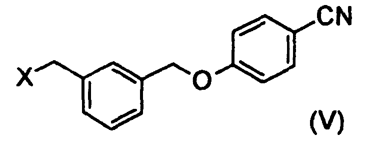 als solche, worin X die vorstehend genannten Bedeutungen, besonders bevorzugt die Bedeutung Hydroxy oder Chlor haben kann.

Weiterhin ist zur erfindungsgemäßen Synthese der Verbindungen der Formel (I) eine der Ausgangsverbindungen, die Verbindung der Formel (VII) von besonderer Bedeutung. Dementsprechend zielt ein weiterer Aspekt der vorliegenden Erfindung auf die Verbindungen der Formel (VII) 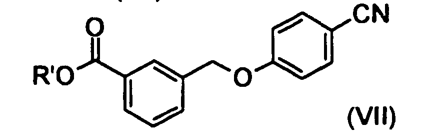 als solche, worin R' C₁-C₄-Alkyl, bevorzugt Methyl oder Ethyl, besonders bevorzugt Methyl bedeutet.

Zur Durchführung des erfindungsgemäßen Herstellverfahrens der Verbindungen der Formel (I) ausgehend von den Nitrilen der Formel (II) wird vorzugsweise wie folgt vorgegangen:

In eine Lösung des Alkalimetall-hexaalkyldisilazans, bevorzugt Lithium-bis(trimethylsilyl)-amid, Natrium-bis(trimethylsilyl)-amid, besonders bevorzugt Lithium-bis(trimethylsilyl)-amid in einem etherischen oder aromatischen organischen Lösemittel, bevorzugt in einem Lösemittel ausgewählt aus der Gruppe Tetrahydrofuran, Toluol, Dioxan, besonders bevorzugt in Tetrahydrofuran oder Dioxan, höchst bevorzugt in Tetrahydrofuran, wird eine Verbindung der Formel (II), vorzugsweise unter Kühlung, besonders bevorzugt bei einer Temperatur zwischen - 50° C und 30° C, besonders bevorzugt bei -20° C bis 10° C, höchst bevorzugt bei ca. 0° C, langsam zudosiert. Die Menge des eingesetzten Alkalimetall-hexaalkyldisilazans bestimmt sich nach der Menge des eingesetzten Nitrils der Formel (II). Pro Mol Nitril der Formel (II) werden mindestens 1 Mol, vorzugsweise 1.01 bis 1.15 Mol Alkalimetall-hexaalkyldisilazan eingesetzt. Die Menge an eingesetztem etherischen Lösemittel beträgt zwischen 0.7 und 1.5, bevorzugt 0.9 bis 1.3 Kg pro mol eingesetzte Verbindung der Formel (II).

Nach beendeter Zugabe der Verbindung der Formel (II) wird die so erhaltene Suspension bei konstanter Temperatur, gegebenenfalls bei einer Temperatur von bis zu 40°C, bevorzugt bei ca. 20-25°C über einen Zeitraum von 6 bis 24 Stunden, vorzugsweise von 8 bis 18 Stunden. bevorzugt 10 bis 12 Stunden gerührt. Dabei kann gegebenenfalls beobachtet werden, daß der zunächst suspendierte Feststoff in Lösung geht.

Sodann kann der Ansatz gegebenenfalls entweder mit weiterem etherischen Lösemittel oder mit einem unpolaren organischen Lösemittel, vorzugsweise mit einem aromatischen organischen Lösemittel verdünnt werden. Bevorzugt wird ein Lösemittel ausgewählt aus der Gruppe bestehend aus Toluol, Benzol, Cyclohexan, Methylcyclohexan oder Xylol eingesetzt, von denen Toluol und Xylol besonders bevorzugt, Toluol höchst bevorzugt ist. Wird der Ansatz verdünnt, ist die Zugabe von bis zu 0.5 L, bevorzugt bis zu 0.3 L Lösemittel pro Mol eingesetzte Verbindung der Formel (II) ausreichend.

Die Reaktion wird vor der Zugabe der Verbindung der Formel (III) auf eine Reaktionstemperatur von -50° C und 20° C, besonders bevorzugt auf -20° C bis 10° C, höchst bevorzugt auf -10 bis 0° C gebracht. Sodann wird die Verbindung der Formel (III) zugesetzt, wobei pro Mol eingesetzte Verbindung (II) mindestens 1 Mol, vorzugsweise 1,05 bis 1,3 Mol besonders bevorzugt 1,1 bis 1,2 Mol der Verbindung der Formel (III) zugesetzt werden.

Nach vollständigem Umsatz wird durch Zugabe einer Säure der Formel HX, vorzugsweise einer anorganischen oder organischen Säure wie zum Beispiel Salzsäure, Schwefelsäure, Phosphorsäure, Essigsäure, Trifluoressigsäure, Oxalsäure und Fumarsäure, insbesondere von wässriger Salzsäure hydrolisiert. Pro Mol ursprünglich eingesetzter Verbindung der Formel (11) wird ca. 1 Mol Säure, insbesondere Salzsäure eingesetzt. Erfindungsgemäß bevorzugt ist die Zugabe von verdünnter Salzsäure (bevorzugt 8-15-%ig, besonders bevorzugt 10-12-%ig). Nach einem Zeitraum von etwa 10 Minuten bis 1 Stunde wird die wässrige Unterphase abgetrennt und in die organische Phase ein organisches Lösemittel, ausgewählt aus der Gruppe Aceton, Methylisobutylketon, Methylethylketon gegebenenfalls auch ein Gemisch aus zweien der vorstehend genannten Lösemittel, besonders bevorzugt ein Gemisch aus Aceton und Methylisobutylketon im Verhältnis 3-1 : 1, besonders bevorzugt im Verhältnis von 2,5-1,5: 1 eingetragen. Durch Zugabe von wässriger Salzsäure wird die Kristallisation der Verbindungen der Formel (IVA) eingeleitet. Pro Mol ursprünglich eingesetzter Verbindung der Formel (II) werden ca. 1 bis 1.2 Mol Säure, vorzugsweise Salzsäure eingesetzt. Erfindungsgemäß bevorzugt ist die Zugabe von bevorzugt 32-37-%iger, besonders bevorzugt 37-%iger Salzsäure. Die Verbindungen der Formel (IVA) werden nach konventionellen Verfahren, beispielsweise durch Zentrifugieren, aus dem Reaktionsgemisch abgetrennt, mit einem organischen Lösemittel, ausgewählt aus der Gruppe Aceton, Methylisobutylketon, Methylethylketon oder einem Carbonsäureester, bevorzugt Aceton gewaschen und getrocknet.

Die Freisetzung der Verbindungen der Formel (I) aus den Säureadditionssalzen der Formel (IV), insbesondere aus den Hydrochloriden der Formel (IVA) erfolgt in der Regel unter möglichst neutralen Reaktionsbedingungen mit basischen Reaktionspartnern, vorzugsweise in Gegenwart von Puffersystemen gemäß der nachstehend beschrieben Vorgehensweise erfolgen:

Zu einer Lösung von tri-Natriumcitrat-Dihydrat, Natriumhydroxid, Kaliumhydroxid, Alkali-oder Erdalkalisalzen organischer oder anorganischer schwacher Säuren, bevorzugt tri-Natriumcitrat-Dihydrat, tri-Natriumcitrat oder Natriumhydroxid, besonders bevorzugt tri-Natriumcitrat-Dihydrat in Wasser wird bei 0 bis 40 °C, bevorzugt bei 20 bis 25 °C, insbesondere bei etwa 20 °C ein organisches Lösemittel ausgewählt aus der Gruppe Aceton, Methylisobutylketon, Methylethylketon, Tetrahydrofuran oder einem Carbonsäureester, besonders bevorzugt Aceton und anschließend eine Verbindung der Formel (IV) gegeben. Pro Mol eingesetzte Verbindung der Formel (IV) werden ca. 1 bis 2 Mol, bevorzugt etwa 1,5 Mol des vorgelegten Natrium- oder Kaliumcitrats sowie etwa 1 bis 3 L des vorstehend genannten organischen Lösemittels, bevorzugt ca. 2 L, eingesetzt. Es wird bei konstanter Temperatur über einen Zeitraum von 20 Minuten bis 2 Stunden, vorzugsweise 1 bis 1,2 Stunden gerührt.

Bei Verwendung der starken Basen, wie Natriumhydroxid, kann der Zugabemodus gegebenenfalls umgekehrt werden. Das kristalline Produkt wird beispielsweise mittels Filtration abgetrennt, mit Wasser salzfrei gewaschen, mit dem vorstehend genannten organischen Lösemittel und abschließend getrocknet.

Die Verbindungen der Formel (II) werden, wie bereits erwähnt, durch Umsetzung einer Verbindung der Formel (V) mit einer Verbindung der Formel (VI) zugänglich. Erfindungsgemäß kann dazu wie nachfolgend beschrieben vorgegangen werden. Die Verbindung der Formel (V), in der X Hydroxy bedeutet, wird in einem möglichst aprotisch-polaren organischen Lösemittel, bevorzugt N,N-Dimethylacetamid , Aceton, Methylethylketon, Methylisobutylketon, N-Methylpyrrolidon, N,N-Dimethylformamid, Tetraalkylhamstoff, besonders bevorzugt in N,N-Dimethylacetamid gelöst. Pro Mol Ausgangsverbindung werden an dieser Stelle erfindungsgemäß 0,5 bis 1,0, vorzugsweise etwa 0,7 L Lösemittel eingesetzt. Anschließend wird die so erhalten Lösung auf eine Temperatur von < 10 °C, vorzugsweise auf eine Temperatur zwischen +5 °C und -20 °C, besonders bevorzugt auf ca. -10 °C bis 0 °C gekühlt. Sodann werden nacheinander ein geeignet substituiertes Sulfonsäurechlorid, gegebenenfalls vorstehend genanntes organisches Lösemittel, eine organische Base, gegebenenfalls vorstehend genanntes organisches Lösemittel sowie die wässrige Lösung einer anorganischen Base zugesetzt. Als geeignet substituiertes Sulfonsäurechlorid kommen erfindungsgemäß Methansulfonsäurechlorid, para-Toluolsulfonsäurechlorid, Benzolsulfonsäurechlorid oder auch Trifluormethansulfonsäurechlorid in Betracht. Vorzugsweise wird Methansulfonsäurechlorid verwendet. Als organische Basen können beispielsweise Dimethylaminopyridin, Pyridin, Methylpyridine, tert. Amine, wie beispielsweise Trimethylamin, Triethylamin, Diisopropylethylamin, oder auch cylische Amine wie N-Methylpyrrolidin oder DBU (Diazabicycloundecen) Verwendung finden. Bevorzugt werden als organische Amine N-Methylpyrrolidin, Trimethylamin, Triethylamin oder Diisopropylethylamin, besonders bevorzugt Triethylamin verwendet. Die organische Base wird wenigstens in stöchiometrischer Menge, bezogen auf die Ausgangsverbindung der Formel (V) eingesetzt. Bevorzugt wird die organische Base in einem Überschuß 10-50 Mol-%, besonders bevorzugt in etwa 30%-igem Überschuß bezogen auf eingesetzte Verbindung der Formel (V) zugegeben. Als wässrige Lösung einer organischen Base gelangen üblicherweise die Lösungen der Hydroxide der Erdalkali- oder Alkalimetalle zur Anwendung, wobei die der Alkalimetalle bevorzugt sind. Die wässrigen Lösungen des Kalium-Hydroxids und des Natriumhydroxids sind erfindungsgemäß von besonderer Bedeutung. Üblicherweise werden 20-50%-ige Lösungen der vorstehend genannten anorganischen Hydroxide verwendet. Konzentriertere Lösungen wie beispielsweise 45%-ige Lösungen sind erfindungsgemäß bevorzugt. Bezogen auf die eingesetzte Verbindung der Formel (V) wird die anorganische Base wenigstens in stöchiometrischer Menge, bevorzugt allerdings in einem Überschuß von 50-100 Mol-% zugesetzt. Besonders bevorzugt wird die anorganische Base in einem Überschuß von etwa 75 Mol-% bezogen auf eingesetzte Verbindung der Formel (V) zugegeben.

Gegebenenfalls kann die Reaktionsmischung jeweils nach der Zugabe des geeignet substituierten Sulfonsäurechlorids bzw. der organischen Base durch Zugabe des vorstehend genannten organischen Lösemittels verdünnt werden. In diesem Fall werden jeweils 2-10 %, bevorzugt etwa 5 % der eingangs vorgelegten Lösemittelmenge zugesetzt.

In jedem Fall wird die Reaktionsmischung nach beendeter Zugabe der wässrigen Lösung der anorganischen Base mit dem eingangs genannten organischen Lösemittel verdünnt. Dabei werden pro Mol eingesetzte Ausgangsverbindung der Formel (V) etwa 0,5 bis 1,0 L, vorzugsweise zwischen 0,7 und 0,8 L des verwendeten Lösemittels zugesetzt. Sodann erfolgt die Zugabe von Alkoholaten bzw. Metallsalzen der Formel (VI). Hierbei kommen bevorzugt die von den Verbindungen der Formel (VI) ableitbaren Natrium- und Kaliumphenolate zurAnwendung. Erfindungsgemäß können bezogen auf eingesetztes Edukt der Formel (V) stöchiometrische Mengen, gegebenenfalls auch substöchiometrische Mengen oder ein Überschuß an Verbindung (VI) zugegeben werden. Nach beendeter Zugabe der Verbindung (VI) wird die Reaktion über einen Zeitraum von etwa 1 bis 3 Stunden, bevorzugt ca. 1,5 bis 2 Stunden bei einer Temperatur von 5 bis 35° C, bevorzugt bei etwa 25 °C geführt und abschließend für einen Zeitraum von etwa 1-3 Stunden, bevorzugt etwa 1,5 bis 2 Stunden bei einer Temperatur von 50 bis 100 °C, bevorzugt bei etwa 70 bis 90 °C nachgerührt. Nach Abschluß der Reaktion wird das Produkt der Formel (II) durch Zugabe eines geeigneten polaren Lösemittels ausgewählt aus der Gruppe der niederen Alkohole und Wasser kristallisiert.

Um Produkte besonderer Reinheit in hoher Ausbeute zu erhalten, hat es sich als erfindungsgemäß bevorzugt erwiesen, wenn zur Kristallisation ein Lösemittelgemisch bestehend aus einem unpolaren organischen Lösemittel, vorzugsweise Xylol oder Toluol, besonders bevorzugt Toluol, einem polaren organischen Lösemittel, vorzugsweise einem niederen Alkohol wie Methanol, Ethanol , Butanol oder Isopropanol, besonders bevorzugt Isopropanol und Wasser zugesetzt wird. Das Volumenverhältnis von unpolarem zu polarem organischen Lösemittel zu Wasser kann variieren in einem Bereich von 1 : 7 -10 : 5 - 8, bevorzugt 1 : 8 - 9 : 6 - 7. Durch Abkühlen auf unter 50 °C, vorzugsweise auf etwa 30 °C wird die Kristallisation des Produkts der Formel (II) vervollständigt. Das kristallisierte Produkt wird nach Isolierung gegebenenfalls mit dem vorstehend genannten niederen Alkohol und mit Wasser gewaschen.

Sollen die Verbindungen der Formel (II) ausgehend von den Verbindungen der Formel (V) in denen X eine andere Bedeutung als Hydroxy aufweist erhalten werden, kann erfindungsgemäß wie folgt vorgegangen werden.

Das von den Verbindungen der Formel (VI) abgeleitete Natrium- oder Kaliumphenolat wird gemeinsam mit einer Verbindung der Formel (V) in Wasser aufgenommen, einem unpolaren organischen Lösemittel versetzt und gegebenfalls unter Phasentransferbedingungen umgesetzt. Als Phasentransferkatalysatoren kommen erfindungsgemäß in Betracht die Gruppe der quarteren Ammoniumsalze, bevorzugt die Halogenide, Sulfate oder Hydroxide von Tetradecyltrimethylammonium-, Hexadecyltrimethylammonium-, Tetrabutylammonium-, Tributylmethylammonium- oder Triethylbenzylammonium-Als unpolares organisches Lösemittel können chlorierte Kohlenwasserstoffe wie Methylenchlorid oder erfindungsgemäß bevorzugt aromatische Kohlenwasserstoffe wie Benzol, Toluol, Xylol, bevorzugt Toluol eingesetzt werden. Die Verbindungen der Formel (V) und (VI) werden in annähernd stöchiometrischem Verhältnis eingesetzt, gegebenfalls kann eine der beiden Reaktanden auch im leichten Überschuß (z.B. 15 %) eingesetzt werden. Die zu verwendende Lösemittelmenge richtet sich nach der Menge eingesetzten Edukts. Pro Mol eingesetzte Verbindung der Formel (VI) werden zwischen 1 und 2 L Wasser und zwischen 0,3 und 1,0 L des organischen Lösemittels, bevorzugt zwischen 1,5 und 1,8 L Wasser und 0,5 bis 0,7 L des organischen Lösemittels eingesetzt.

Die Reaktion wird unter intensivem Rühren über einen Zeitraum von 3 bis 9, bevorzugt 5 bis 7 Stunden bei einer Temperatur von 50 bis 100 °C bevorzugt bei 70 bis 80 °C geführt. Anschließend wird zur Kristallisation des Produkts in die abgetrennte organische Phase ein polares organisches Lösemittel, vorzugsweise ein niederer Alkohol, besonders bevorzugt Isopropanol eingebracht. Durch Abkühlen auf unter 50 °C, vorzugsweise auf etwa 30 °C wird die Kristallisation des Produkts der Formel (II) vervollständigt. Das kristallisierte Produkt der Formel (II) wird nach Isolierung gegebenenfalls mit dem vorstehend genannten niederen Alkohol und mit Wasser gewaschen.

Die Herstellung der, wie bereits erwähnt, einen Aspekt der vorliegenden Erfindung betreffenden Ausgangsverbindungen der Formel (V) 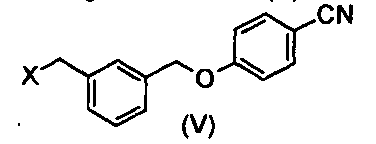 kann in Analogie zu an sich bekannten Syntheseverfahren erfolgen. Die Herstellung der Verbindung der Formel (V) in der X Hydroxy bedeutet, kann durch Umsetzung von beispielsweise 3-Halogenmethyl-benzoesäuremethylestern mit 4-Hydroxybenzonitril im Sinne einer Wilkinson-Ethersynthese erfolgen. Der dadurch erhaltene 3-(4-Cyano-phenoxy)-benzoesäuremethylester (VII) ist unter analoger Anwendung gängiger Standardverfahren reduktiv in die Verbindung der allgemeinen Formel (V) mit X=Hydroxy (= 4-(3-HydroXymethyl-benzyloxy)-benzonitril) überführbar.

Die Verbindungen der Formel (V) in denen X Chlor oder Brom bedeutet, können in Analogie zu an sich bekannten Syntheseverfahren aus der Verbindung der Formel (V), in der X für Hydroxy steht, unter Einsatz gängiger Halogenierungsreagenzien wie beispielsweise Thionylchlorid, Phosphoroxychlorid oder Phosphorpentachlorid, Methansulfonsäurechlorid, Benzolsulfochlorid, bevorzugt Thionylchlorid oder Methansulfonsäurechlorid dargestellt werden.

Die Verbindungen der Formel (V) in denen X Mesylat, Triflat oder Tosylat bedeutet, können in Analogie zu an sich bekannten Syntheseverfahren aus der Verbindung der Formel (V), in der X für Hydroxy steht, durch Umsetzung mit den jeweiligen Sulfonsäurechloriden in aprotischen, vorzugsweise polaren organischen Lösemitteln, vorzugsweise ausgewählt aus der Gruppe, Dichlormethan, N,N-Dimethylacetamid, Dimethylformamid, Acetonitril, N-Methylpyrrolidon, Tetraalkylhamstoff in Gegenwart organischer Basen, vorzugsweise ausgewählt aus der Gruppe Dimethylaminopyridin, Pyridin, Methylpyridin, N-Methylpyrrolidin, Trimethylamin, Triethylamin, Diisopropylethylamin und DBU (Diazabicycloundecen) erhalten werden.

Die nachfolgenden Beispiele dienen der Illustration exemplarisch durchgeführter, erfindungsgemäßer Syntheseverfahren zur Herstellung der Verbindung der Formel (I). Sie sind lediglich als mögliche, beispielhaft dargestellte Vorgehensweisen zu verstehen, ohne die Erfindung auf deren Inhalt zu beschränken.

### Beispiel 1 : 3-(4-Cyano-phenoxymethyl)-benzoesäuremethylester:

10,00 kg (43,6 mol) 3-(Brommethyl)benzoesäuremethylester und 5,21 kg (43,74 mol) 4-Hydroxybenzonitril werden in 100 L Aceton gelöst und mit 8,4 kg (60,7 mol) Kaliumcarbonat in Gegenwart von 0,1 kg Natriumiodid ca 4 h unter Rückflußbedingungen gerührt. Danach werden 35 L Aceton abdestilliert und bei Rückflußbedingungen 100 L Wasser eingetragen. Das Reaktionsgemisch wird auf 20 °C abgekühlt und die Kristallisation durch Einbringen von weiteren 30 L Wassser vervollständigt. Die entstandenen Kristalle werden abgetrennt, mit 50 L Wasser gewaschen und im Vakuum getrocknet.
Ausbeute: 11,1 kg (95%) 3-(4-Cyano-phenoxymethyl)-benzoesäuremethylester; Schmelzpunkt 109...112 °C, weißer Feststoff,
DC (Kieselgel 60 F254 -Fertigplatte (Merck): Rf=0,5 (Toluol : Aceton=9:1)

### Beispiel 2: 4-(3-Hydroxymethyl-benzyloxy)-benzonitril:

20,05 kg (26,7 mol) 3-(4-Cyano-phenoxymethyl)-benzoesäuremethylester werden in 100 L THF und 40 L Methanol gelöst. Bei 40 bis 45 °C werden 8,51 kg Natriumboranat portionsweise eingetragen. Die Reaktion wird durch Nachrühren der Reaktionsmischung auf 61 bis 63 °C über ca. 5 Stunden vervollständigt. Das Reaktionsgemisch wird danach auf 25 °C abgekühlt und 90 L einer 15%igen Natronlauge eingetragen. Nach dem Ausrühren wird die wässrige Oberphase abgetrennt und mit 30 L einer 22,5-%igen Natronlauge versetzt. Nach dem Ausrühren wird die wässrige Oberphase abgetrennt und von dieser ca. 100 L Lösungsmittel bei 63 bis 75 °C Sumpftemperatur abdestilliert. Der Destillationsrückstand wird durch Einbringen von 20 L Isopropanol bei 50 bis 60 °C und und 150 L Wasser bei 40 bis 50°C zur Kristallisation gebracht. Nach Abkühlen der Suspension auf 20 bis 30 °C werden die Kristalle abgetrennt, mit 60 bis 100 L Wasser und portionsweise mit 25 L kaltem Isopropanol gewaschen und im Vakuum getrocknet.
Ausbeute: 15,8 kg (88%) 4-(3-Hydroxymethyl-benzyloxy)-benzonitril;
Schmelzpunkt(DSC): 110-115 °C, weißer Feststoff
IR: 3444 /cm (OH-Bande); 2229 /cm (CN-Bande)

### Beispiel 3: 4-(3-Chlormethyl-benzyloxy)-benzonitril:

### Variante A:

7,18 g (30 mmol) 4-(3-Hydroxymethyl-benzyloxy)-benzonitril werden in 80 ml Dichlormethan gelöst, mit 4.13g (35 mmol) Thionylchlorid und 0.1g DMF versetzt und bis zum Ende der Gasentwicklung unter Erwärmen auf 40 °C gerührt. Nach Abkühlen wird die organische Reaktionsmischung nacheinander mit Wasser und verdünnter Natronlauge gewaschen und durch Einengen zur Kristallisation gebracht. Ausbeute: 6,8g (88%) 4-(3-Chlormethyl-benzyloxy)-benzonitril;
DC (Kieselgel 60 F254 -Fertigplatte (Merck): Rf= 0,9 (Toluol-Aceton = 9:1),
Rf = 0,44 (Toluol)

### Variante B:

7,18 g (30 mmol) 4-(3-Hydroxymethyl-benzyloxy)-benzonitril werden in 22 ml N,N-Dimethylacetamid gelöst, mit 4.47g (39 mmol) Methansulfonsäurechlorid und 3,95g (39mmol) Triethylamin versetzt und bei 20-30 °C 10 Stunden gerührt. Anschließend wird das ausgefallene Triethylammoniumchlorid abfiltriert, das Filtrat mit 30ml Isopropanol versetzt und das gewünschte 4-(3-Chlormethyl-benzyloxy)-benzonitrile durch Eindosieren von 30 ml Wasser zur Kristallisation gebracht. Die Suspension wird 15 min bei 10°C nachgerührt und filtriert. Die Kristalle werden mit einer Mischung aus 5 ml Isopropanol und 20 ml Wasser nachgewaschen und bei 20°C im Vakuum getrocknet.
Ausbeute: 6,8g (88%) 4-(3-Chlormethyl-benzyloxy)-benzonitrite
Schmelzpunkt: 65-68 °C

### Beispiel 4: Natrium-4{1-methyl-1-[4-tetrahydro-pyran-2-yloxy)-phenyl]-ethyl}-phenolat

121,8 kg Bisphenol A werden in 480 l Toluol und 46 l THF suspendiert. Nach Einbringen des Katalysators (1,3 kg 37%-ige Salzsäure) werden 44,9 kg 3,4-Dihydro-2H-pyran so eindosiert, daß 40°C nicht überschritten werden. Dabei geht der Feststoff in Lösung. Anschließend wird die Reaktionsmischung mit 26, 4 kg 45%iger Natronlauge und 260 l Wasser versetzt. Die organische Oberphase wird abgetrennt und es werden ca. 50 l Lösungsmittel destillativ entfernt.
Bei 30 bis 40 °C wird die organische Phase mehrmals mit verdünnter Natronlauge so gewaschen, daß eine ausreichende Reinheit (DC-Kontrolle) erzielt werden kann. Wenn die wässrige Unterphase im pH-Bereich von 11,8 bis 12,2 liegt, kann überschüssiges Bisphenol A gut abgetrennt werden.
Die extraktiv gereinigte toluolische Phase wird mit 11 l Isopropanol und 80 l Wasser versetzt und auf 50 bis 55°C erwärmt. Durch Einbringen von 47,4 kg 45%iger Natronlauge und Abkühlen der Reaktionsmischung auf 20 bis 25 °C erhält man eine Kristallsuspension. Die Kristalle werden mittels Filtration abgetrennt, mit ca. 160 l Toluol nachgewaschen und anschließend im Vakuum getrocknet.
Ausbeute: 96,5 kg (54%) (Als Tetra-Hydrat)

### Beispiel 5: Synthese von 4-[3-(4-{1-Methyl-1-[4-(tetrahydro-pyran-2-yloxy)-phenyl]-ethyl}-phenoxymethyl)-benzyloxy]-benzonitril:

### Variante A: (ausgehend von Beispiel 2)

Zu einer Lösung von 45 kg (188mol) 4-(3-Hydroxymethyl-benzyloxy)-benzonitril (Beispiel 2) in 133 L N,N-Dimethylacetamid dosiert man bei ca. -10 bis 0 °C nacheinander ein: 28 kg (244 mol) Methansulfonsäurechlorid , 6 L N,N-Dimethylacetamid, 24,7 kg (244 mol) Triethylamin, 6 L N,N-Dimethylacetamid, 29,4 kg 45%ige Natronlauge, 143 L N,N-Dimethylacetamid, 59,7 kg (178,5 mol) Beispiel 4 (≡ Natrium-4-{1-methyl-1-[4-tetrahydro-pyran-2-yloxy)-phenyl]-ethyl}-phenolat, als Tetrahydrat). Danach wird die Reaktionsmischung 2 h bei 25 °C und weitere 1,5 h bei 75 bis 80 °C nachgerührt. Nach Zugabe von 32 L Toluol, 255 L Isopropanol und 200 L Wasser setzt die Kristallisation ein, die durch Abkühlen auf 30 °C vervollständigt wird. Das kristalline Produkt wird mittels Filtration abgetrennt , mit Isopropanol und Wasser nachgewaschen und anschließend im Vakuum getrocknet. Ausbeute: 85 kg (90%) 4-[3-(4-{1-Methyl-1-[4-(tetrahydro-pyran-2-yloxy)-phenyl]-ethyl}-phenoxymethyl)-benzyloxy]-benzonitril;

### Variante B: (ausgehend von Beispiel 3)

19.4 kg (50 mol) Beispiel 4 (≡ Natrium-4-{1-methyl-1-[4-tetrahydro-pyran-2-yloxy)-phenyl]-ethyl}-phenolat, als Tetrahydrat) und 12,2 kg 47,5 mol) Beispiel 3 (≡ 4-(3-Chloromethyl-benzyloxy)-benzonitril) werden mit 85 L Wasser, einem Phasentransferkatalysator (z.B.: 2,1 kg (2,5 mol) einer 40%igen wässrigen Lösung aus Tetradecyltrimethylammoniumbromid und 32 L Toluol versetzt und 6 h bei ca. 80°C intensiv gerührt. Danach werden in die bei 50 bis 70 °C abgetrennte organische Oberphase 44 L Isopropanol eindosiert, die erhaltene Kristallsuspension auf ca. 25°C abgekühlt und filtriert. Die abgetrennten Kristalle werden zweimal mit je 25 L kaltem Isopropanol gewaschen und im Vakuum getrocknet.
Ausbeute: 22,8 kg (90%) 4-[3-(4-{1-Methyl-1-[4-(tetrahydro-pyran-2-yloxy)-phenyl]-ethyl}-phenoxymethyl)-benzyloxy]-benzonitril;

### Beispiel 6: {[4-(3-[4-[1-(4-Hydroxy-phenyl)-1-methyl-ethyl]-phenoxymethyl}-benzyloxy)-phenyl]-imino-methyl}-carbaminsäureethylester-Hydrochlorid

In eine Lösung von 45,5 kg (272mol) Lithium-bis(trimethylsilyl-)amid in 266 kg THF werden 132 kg (247 mol) 4-(3-{4-[1-(4-Tetrahydropyranyl-phenyl)-1-methyl-ethyl]-phenoxymethyl}-benzyloxy)-benzonitrile (Beispiel 5) bei ca. 0 °C eindosiert. Die erhaltene Suspension wird ca. 10 h bei ca. 25°C gerührt. Dabei geht der Feststoff in Lösung. Nach Zugabe von 68 L Toluol wird die Reaktionsmischung auf -10 ° bis 0 °C gekühlt und bei dieser Temperatur werden 30,8 kg (284mol) Chlorameisensäureethylester in das Reaktionsgefäß eingebracht. Nach Ablauf der vollständigen Reaktion werden 24,3 kg 37%-ige Salzsäure (verdünnt mit 50 L Wasser) eindosiert und ca. 20 min später die wässrige Unterphase abgetrennt. Durch nachfolgendes Einbringen von 106 L Aceton, 48 L Methylisobutylketon und 24,3 kg 37%-ige Salzsäure wird die Kristallisation des Zielproduktes eingeleitet. Man erhält 123 kg (87%) {[4-(3-{4-[1-(4-Hydroxy-phenyl)-1-methyl-ethyl]-phenoxymethyl}-benzyloxy)-phenyl]-imino-methyl}-carbaminsäureethylester-Hydrochlorid nach Abzentrifugieren, Waschen mit Aceton und Trocknen im Vakuum.
Schmelzpunkt: 170-175 °C

### Beispiel 7: {[4-(3-{4-[1-(4-Hydroxy-phenyl)-1-methyl-ethyl]-phenoxymethyl}-benzyloxy)-phenyl]-imino-methyl}-carbaminsäureethylester

Zu einer Lösung von 109 kg tri-Natriumcitrat-Dihydrat werden bei 20 °C 466 L Aceton und 142 kg {[4-(3-{4-[1-(4-Hydroxy-phenyl)-1-methyl-ethyl]-phenoxymethyl}-benzyloxy)-phenyl]-imino-methyl}-carbaminsäureethylester-Hydrochlorid (Beispiel 6) gegeben. Nach einer Stunde Rühren wird das kristalline Produkt mittels Filtration abgetrennt, mit Wasser salzfrei gewaschen, mit ca. 100 L Aceton nachgewaschen und abschließend im Vakuum getrocknet.
Man erhält 116 kg (90%) {[4-(3-{4-[1-(4-Hydroxy-phenyl)-1-methyl-ethyl]-phenoxymethyl}-benzyloxy)-phenyl]-imino-methyl}-carbaminsäureethylester.

## Patentansprüche

1. Verfahren zur Herstellung von Verbindungen der Formel (I) 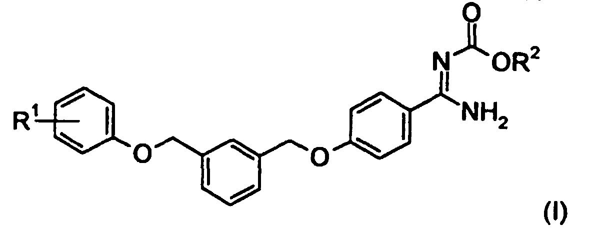 worin
R¹ ein Rest ausgewählt aus der Gruppe bestehend aus Methyl, Ethyl, Propyl, Cyclopentyl, Cyclohexyl, Phenyl, Benzyl und -C(Me₂)Phenyl bedeutet, der jeweils ein zwei oder dreifach durch Hydroxy substituiert sein kann;
R² ein Rest ausgewählt aus der Gruppe bestehend aus Methyl, Ethyl, Propyl und Benzyl bedeutet,
**dadurch gekennzeichnet, daß** eine Verbindung der Formel (II) 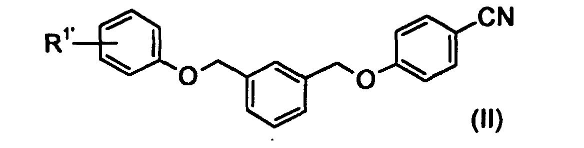 worin
R^{1'} ein Rest ausgewählt aus der Gruppe bestehend aus Methyl, Ethyl, Propyl, Cyclopentyl, Cyclohexyl, Phenyl, Benzyl und -C(Me₂)Phenyl bedeutet, der jeweils ein zwei oder dreifach durch eine Gruppe -O-PG substituiert sein kann, wobei die Gruppe -O-PG für eine geschützte Hydroxylfunktion ausgewählt aus der Gruppe bestehend aus Methoxymethyloxy, 2-Methoxyethoxymethyloxy, 1-Ethoxyethyloxy, 2-Tetrahydropyranyloxy , 1-Butoxyethyloxy, tert.-Butyloxy, Benzyloxy und 4-Methoxybenzyloxy steht,
zunächst in einem etherischen oder aromatischen Lösemittel mit einem Alkalimetall-Hexaalkyldisilazan umgesetzt und anschließend mit einer Verbindung der Formel (III)
R²-O-COX' (III)
worin
R² die vorstehend genannte Bedeutung aufweist und
X' Chlor, Brom oder -O-R² bedeutet, behandelt wird,
nach Aufarbeitung mittels einer Säure der Formel HY eine Verbindung der Formel (IV) und/oder ihrer tautomeren Form 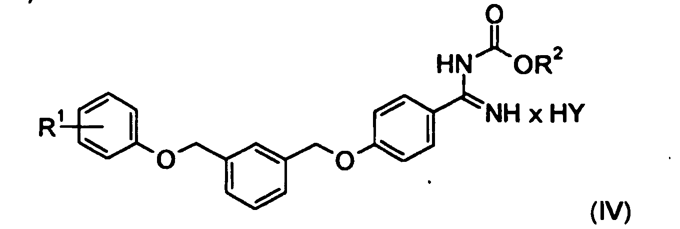 worin die Reste R¹ und R² die vorstehend genannte Bedeutung aufweisen, und Y einen beliebigen Säurerest bedeutet,
isoliert wird, und aus dieser die Verbindung der Formel (I) freigesetzt wird.

2. Verfahren nach Anspruch 1, worin
R¹ ein Rest ausgewählt aus der Gruppe bestehend aus Phenyl, Benzyl und -C(Me₂)Phenyl bedeutet, der jeweils ein oder zweifach durch Hydroxy substituiert sein kann;
R² ein Rest ausgewählt aus der Gruppe bestehend aus Ethyl, Propyl und Benzyl bedeutet,
**dadurch gekennzeichnet, daß** eine Verbindung der Formel (II), worin
R^{1'} ein Rest ausgewählt aus der Gruppe bestehend aus Phenyl, Benzyl und -C(Me₂)Phenyl bedeutet, der jeweils ein oder zweifach durch eine Gruppe -O-PG substituiert sein kann,
wobei die Gruppe -O-PG für eine geschützte Hydroxylfunktion ausgewählt aus der Gruppe bestehend aus Methoxymethyloxy, 2-Methoxyethoxymethyloxy, 1-Ethoxyethyloxy, 2-Tetrahydropyranyloxy , 1-Butoxyethyloxy, tert.-Butyloxy, Benzyloxy und 4-Methoxybenzyloxy, steht,
zunächst in einem etherischen oder aromatischen Lösemittel mit einem Alkalimetall-Hexaalkyldisilazan umgesetzt und anschließend mit einer Verbindung der Formel (III)
R²-O-COX' (III)
worin
R² die vorstehend genannte Bedeutung aufweist und
X' Chlor, Brom oder -O-R² bedeutet, behandelt wird,
nach Aufarbeitung mittels wässriger Salzsäure eine Verbindung der Formel (IVA) 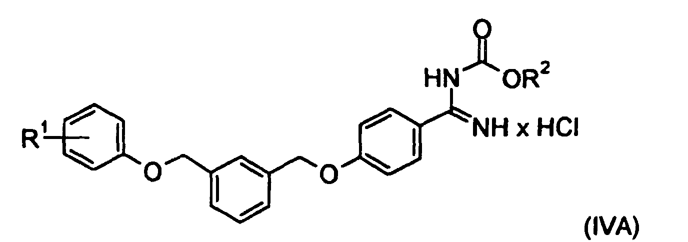 worin die Reste R¹ und R² die vorstehend genannte Bedeutung aufweisen, isoliert wird und aus dieser die Verbindung der Formel (I) freigesetzt wird.

3. Verfahren nach Anspruch 1 oder 2,
worin
R¹ -C(Me₂)Phenyl bedeutet, das gegebenenfalls einfach durch Hydroxy substituiert sein kann und
R² Ethyl bedeutet,
**dadurch gekennzeichnet, daß** eine Verbindung der Formel (II), worin
R^{1'} -C(Me₂)Phenyl bedeutet, das gegebenenfalls einfach durch eine Gruppe -O-PG substituiert sein kann, wobei die Gruppe -O-PG für eine geschützte Hydroxylfunktion ausgewählt aus der Gruppe bestehend aus Methoxymethyloxy, 2-Tetrahydropyranyloxy , 1-Butoxyethyloxy, tert.-Butyloxy, Benzyloxy und 4-Methoxybenzyloxy, steht,
zunächst in einem etherischen oder aromatischen Lösemittel mit einem Alkalimetall-Hexaalkyldisilazan umgesetzt und anschließend mit einer Verbindung der Formel (III)
R²-O-COX' (III)
worin
R² die vorstehend genannte Bedeutung aufweist und
X' Chlor, Brom oder -O-R², bedeutet, behandelt wird,
nach Aufarbeitung mittels einer Säure der Formel Hy eine Verbindung der Formel (IV) worin die Reste Y, R¹ und R² die vorstehend genannte Bedeutung aufweisen, isoliert wird und aus dieser die Verbindung der Formel (I) freigesetzt wird.

4. Verfahren zur Herstellung einer Verbindung der Formel I nach einem der Ansprüche 1 bis 3, **gekennzeichnet durch** die folgenden Schritte:
(e) Umsetzung von 3-Halogenmethylbenzoesäure-C₁-C₄-alkylestern mit 4-Hydroxybenzonitril im Sinne einer Wilkinson-Ethersynthese;
(f) reduktive Überführung von 3-(4-Cyano-phenoxy)-benzoesäurealkylestern der Formel (VII) 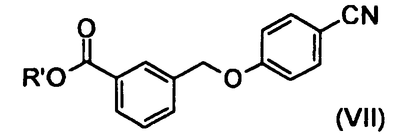 worin R' C₁-C₄-Alkyl bedeutet, in eine Verbindung der Formel (V), 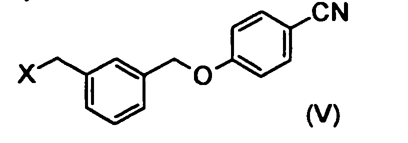 worin X für Hydroxy steht;
(g) gegebenenfalls Behandlung der Verbindung der Formel (V), worin X für Hydroxy steht mit einem Halogenierungsreagenz oder einem Sulfonsäurechlorid;
(h) Umsetzung der Verbindung der Formel (V), worin X Hydroxy, Chlor, Brom, Mesylat, Triflat oder Tosylat bedeutet, mit einem Phenol Derivat der Formel (VI) 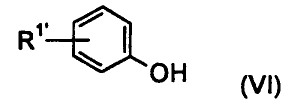 worin R^{1'} die in den Ansprüchen 1 bis 4 genannten Bedeutungen aufweist; gegebenenfalls in Form der entsprechenden Natrium- oder KaliumPhenolate, unter basischen Reaktionsbedingungen in einem polaren organischen Lösemittel;
(i) Überführung der erhaltenen Verbindung der Formel (II) in die Verbindung der Formel (I) nach dem Verfahren gemäß der Ansprüche 1 bis 3.

5. Zwischenprodukte der Formel (IVA) und/oder ihrer tautomeren Form 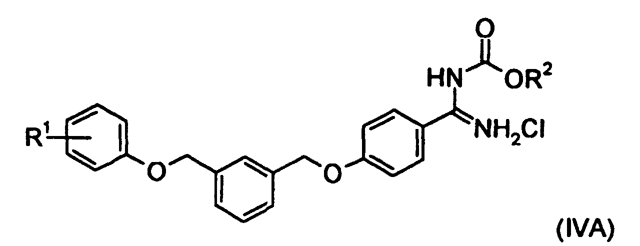 worin die Reste R¹ und R² die in den Ansprüchen 1, 2 oder 3 genannten Bedeutungen haben können.

6. Zwischenprodukte der Formel (V), 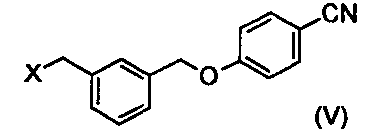 worin X Hydroxy, Chlor, Brom, Mesylat, Triflat oder Tosylat bedeutet.

7. Zwischenprodukte gemäß Anspruch 6, worin X Hydroxy oder Chlor bedeutet.

8. Zwischenprodukte der Formel (VII) 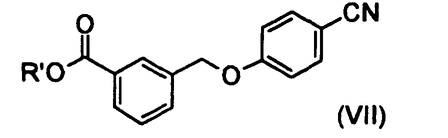 worin R' C₁-C₄-Alkyl bedeutet.

9. Verwendung der Zwischenprodukte der Formel (IV) gemäß Anspruch 5 zur Herstellung einer Verbindung der Formel (I) gemäß Anspruch 1.

10. Verwendung der Zwischenprodukte der Formel (V) gemäß Anspruch 6 oder 7 zur Herstellung einer Verbindung der Formel (I) gemäß Anspruch 1.

11. Verwendung der Zwischenprodukte der Formel (VII) gemäß Anspruch 8 zur Herstellung einer Verbindung der Formel (I) gemäß Anspruch 1.

## Claims

1. Process for preparing compounds of formula (I) 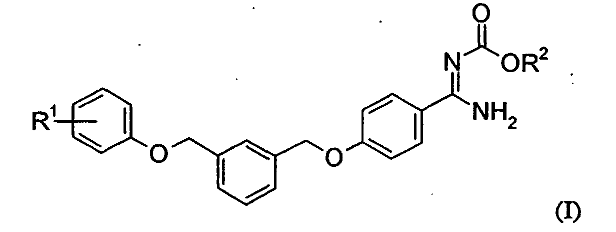 wherein
R¹ denotes a group selected from among methyl, ethyl, propyl, cyclopentyl, cyclohexyl, phenyl, benzyl and -C(Me₂)phenyl, each of which may be mono-, di- or trisubstituted by hydroxy;
R² denotes a group selected from among methyl, ethyl, propyl and benzyl,
**characterised in that** a compound of formula (II) 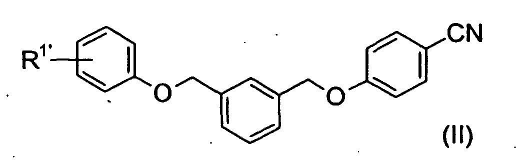 wherein
R^{1'} denotes a group selected from among methyl, ethyl, propyl, cyclopentyl, cyclohexyl, phenyl, benzyl and -C(Me₂)phenyl, each of which may be mono-, di- or trisubstituted by a group -O-PG, the group -O-PG denoting a protected hydroxyl function selected from among methoxymethyloxy, 2-methoxyethoxymethyloxy, 1-ethoxyethyloxy, 2-tetrahydropyranyloxy, 1-butoxyethyloxy, tert.-butyloxy, benzyloxy and 4-methoxybenzyloxy,
is first reacted in an ethereal or aromatic solvent with an alkali metal hexaalkyldisilazane and is then treated with a compound of formula (III)
R²-O-COX' (III)
wherein
R² is as hereinbefore defined and
X' denotes chlorine, bromine or -O-R²,
after working up using an acid of formula HY a compound of formula (IV) 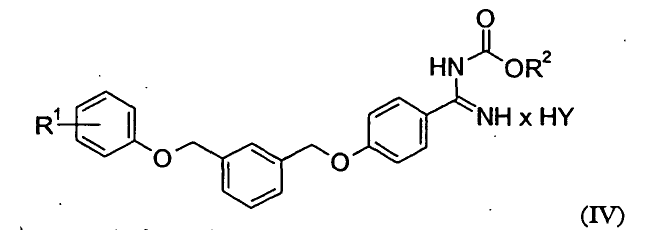 and/or the tautomeric form thereof,
wherein the groups R¹ and R² are as hereinbefore defined and Y denotes any desired acid group,
is isolated and from this the compound of formula (I) is liberated.

2. Process according to claim 1, wherein
R¹ denotes a group selected from among phenyl, benzyl and -C(Me₂)phenyl, each of which may be mono- or disubstituted by hydroxy;
R² denotes a group selected from among ethyl, propyl and benzyl,
**characterised in that** a compound of formula (II)
wherein
R^{1'} denotes a group selected from among phenyl, benzyl and -C(Me₂)phenyl, each of which may be mono- or disubstituted by a group -O-PG, the group -O-PG denoting a protected hydroxyl function selected from among methoxymethyloxy, 2-methoxyethoxymethyloxy, 1-ethoxyethyloxy, 2-tetrahydropyranyloxy , 1-butoxyemyloxy, tert.-butyloxy, benzyloxy and 4-methoxybenzyloxy,
is first reacted in an ethereal or aromatic solvent with an alkali metal hexaalkyldisilazane and then treated with a compound of formula (III)
R²-O-COX' (III)
wherein
R² is as hereinbefore defined and
X' denotes chlorine, bromine or -O-R²,
after working up with aqueous hydrochloric acid a compound of formula (IVA) 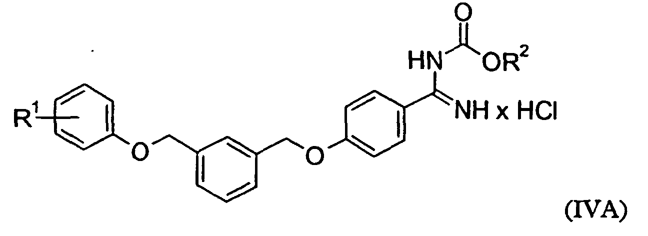 wherein the groups R¹ and R² are as hereinbefore defined is isolated and from this the compound of formula (I) is liberated.

3. Process according to claim 1 or 2,
wherein
R¹ denotes -C(Me₂)phenyl which may optionally be monosubstituted by hydroxy and
R² denotes ethyl,
**characterised in that** a compound of general formula (II)
wherein
R^{1'} denotes -C(Me₂)phenyl, which may optionally be monosubstituted by a group -O-PG, the group -O-PG denoting a protected hydroxyl function selected from among methoxymethyloxy, 2-tetrahydropyranyloxy , 1-butoxyethyloxy, tert.-butyloxy, benzyloxy and 4-methoxybenzyloxy,
is first reacted in an ethereal or aromatic solvent with an alkali metal hexaalkyldisilazane and then treated with a compound of formula (III)
R²-O-COX' (III)
wherein
R² is as hereinbefore defined and
X' denotes chlorine, bromine or -O-R²,
after working up using an acid of formula HY a compound of formula (IV) is isolated wherein the groups Y, R¹ and R² are as hereinbefore defined and from this the compound of formula (I) is liberated.

4. Process for preparing a compound of formula I according to one of claims 1 to 3, **characterised by** the following steps:
(e) reacting C₁₋₄-alkyl 3-halomethylbenzoates with 4-hydroxybenzonitrile in the manner of a Wilkinson ether synthesis;
(f) reductively converting alkyl 3-(4-cyano-phenoxy)benzoates of formula (VII) 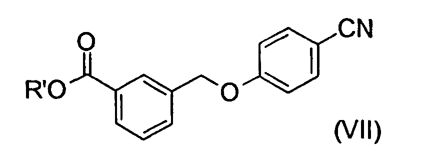 wherein R' denotes C₁₋₄-alkyl, into a compound of formula (V) 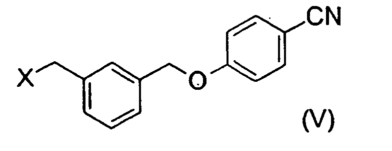 wherein X denotes hydroxy;
(g) optionally treating the compound of formula (V) wherein X denotes hydroxy with a halogenating reagent or a sulphonic acid chloride;
(h) reacting the compound of formula (V) wherein X denotes hydroxy, chlorine, bromine, mesylate, triflate or tosylate, with a phenol derivative of formula (VI) 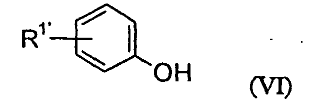 wherein R^{1'} has the meanings given in claims 1 to 4; optionally in the form of the corresponding sodium or potassium phenoxides, under basic reaction conditions, in a polar organic solvent;
(i) converting the resulting compound of formula (II) into the compound of formula (I) using the method according to claims 1 to 3.

5. Intermediate products of formula (IVA) and/or the tautomeric form thereof 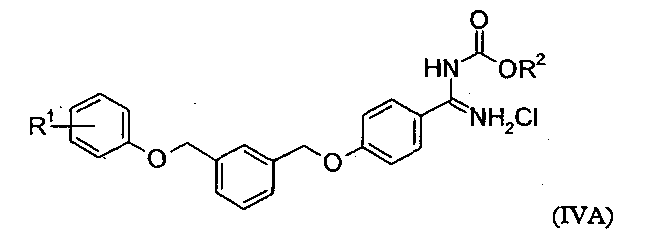 wherein the groups R¹ and R² may have the meanings given in claims 1, 2 or 3.

6. Intermediate products of general formula (V), 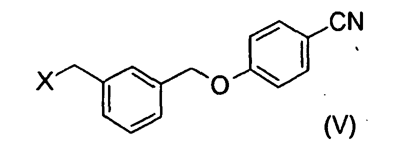 wherein X denotes hydroxy, chlorine, bromine, mesylate, triflate or tosylate.

7. Intermediate products according to claim 6, wherein X denotes hydroxy or chlorine.

8. Intermediate products of general formula (VII) 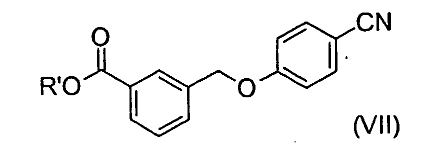 wherein R' denotes C₁₋₄-alkyl.

9. Use of the intermediate products of general formula (IV) according to claim 5 for preparing a compound of formula (I) according to claim 1.

10. Use of the intermediate products of general formula (V) according to claim 6 or 7 for preparing a compound of formula (I) according to claim 1.

11. Use of the intermediate products of general formula (VII) according to claim 8 for preparing a compound of formula (I) according to claim 1.

## Revendications

1. Procédé de production de composés de formule (I) 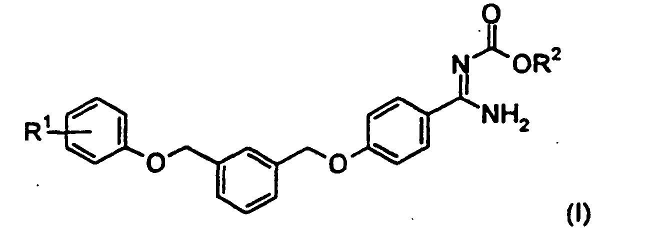 où
R¹ représente un groupement choisi dans le groupe consistant en méthyle, éthyle, propyle, cyclopentyle, cyclohexyle, phényle, benzyle et -C(Me₂)phényle, qui peut être substitué dans chaque cas une, deux ou trois fois par hydroxy ;
R² représente un groupement choisi dans le groupe consistant en méthyle, éthyle, propyle et benzyle,
**caractérisé en ce qu'**un composé de formule (II) 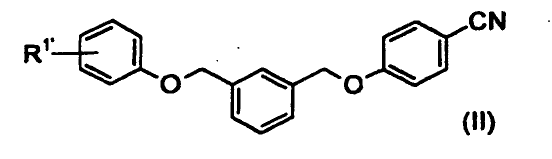 où
R^{1'} représente un groupement choisi dans le groupe consistant en méthyle, éthyle, propyle, cyclopentyle, cyclohexyle, phényle, benzyle et -C(Me₂)phényle, qui peut être substitué dans chaque cas une, deux ou trois fois par un groupe -O-PG, où le groupe -O-PG représente une fonction hydroxyle protégée choisie dans le groupe consistant en méthoxyméthyloxy, 2-méthoxyéthoxyméthyloxy, 1-éthoxyéthyloxy, 2-tétrahydropyranyloxy, 1-butoxyéthyloxy, tert.-butyloxy, benzyloxy et 4-méthoxybenzyloxy,
est d'abord mis à réagir dans un solvant éthéré ou aromatique avec un hexaalkyldisilazane de métal alcalin puis traité avec un composé de formule (III)
R²-O-COX' (III)
où
R² présente la signification citée précédemment et
X' représente le chlore, le brome ou -O-R²,
après une fin de traitement au moyen d'un acide de formule HY un composé de formule (IV) et/ou sa forme tautomère 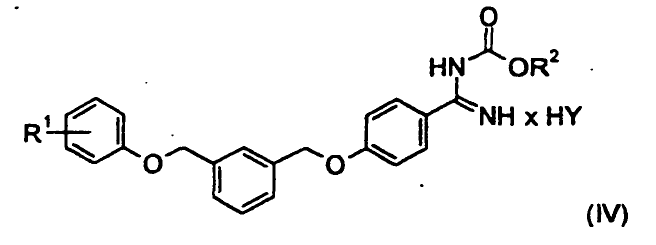 où les groupements R¹ et R² présentent la signification citée précédemment et Y représente un groupement acide quelconque,
est isolé et le composé de formule (I) est libéré à partir de celui-ci.

2. Procédé selon la revendication 1 où
R¹ représente un groupement choisi dans le groupe consistant en phényle, benzyle et -C(Me₂)phényle, qui peut être substitué dans chaque cas une ou deux fois par hydroxy ;
R² représente un groupement choisi dans le groupe consistant en éthyle, propyle et benzyle,
**caractérisé en ce qu'**un composé de formule (II) où
R^{1'} représente un groupement choisi dans le groupe consistant en phényle, benzyle et -C(Me₂)phényle, qui peut être substitué dans chaque cas une ou deux fois par un groupe -O-PG,
où le groupe -O-PG représente une fonction hydroxyle protégée choisie dans le groupe consistant en méthoxyméthyloxy, 2-méthoxyéthoxyméthyloxy, 1-éthoxyéthyloxy, 2-tétrahydropyranyloxy, 1-butoxyéthyloxy, tert.-butyloxy, benzyloxy et 4-méthoxybenzyloxy,
est d'abord mis à réagir dans un solvant éthéré ou aromatique avec un hexaalkyldisilazane de métal alcalin puis traité avec un composé de formule (III)
R²-O-COX' (III)
où
R² présente la signification citée précédemment et
X' représente le chlore, le brome ou -O-R²,
après une fin de traitement au moyen d'acide chlorhydrique aqueux un composé de formule (NA) 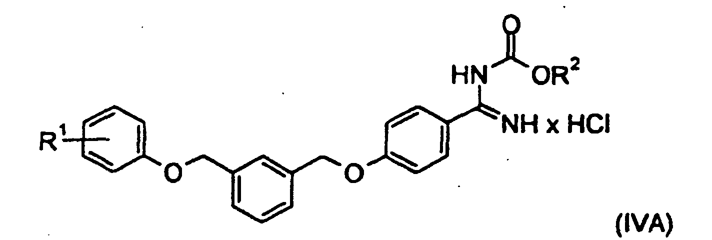 où les groupements R¹ et R² présentent la signification citée précédemment, est isolé et le composé de formule (I) est libéré à partir de celui-ci.

3. Procédé selon la revendication 1 ou 2
où
R¹ représente -C(Me₂)phényle, qui peut éventuellement être substitué une fois par hydroxy et
R² représente éthyle,
**caractérisé en ce qu'**un composé de formule (II) où
R^{1'} représente -C(Me₂)phényle, qui peut éventuellement être substitué une fois par un groupe -O-PG, où le groupe -O-PG représente une fonction hydroxyle protégée choisie dans le groupe consistant en méthoxyméthyloxy, 2-tétrahydropyranyloxy, 1-butoxyéthyloxy, tert.-butyloxy, benzyloxy et 4-méthoxybenzyloxy,
est d'abord mis à réagir dans un solvant éthéré ou aromatique avec un hexaalkyldisilazane de métal alcalin puis traité avec un composé de formule (III)
R²-O-COX' (III)
où
R² présente la signification citée précédemment et
X' représente le chlore, le brome ou -O-R²,
après une fin de traitement au moyen d'un acide de formule HY un composé de formule (IV) où les groupements Y, R¹ et R² présentent la signification citée précédemment, est isolé et le composé de formule (I) est libéré à partir de celui-ci.

4. Procédé de production d'un composé de formule I selon l'une des revendications 1 à 3 **caractérisé par** les étapes suivantes :
(e) réaction de C₁-C₄-alkylesters d'acide 3-halogénométhylbenzoïque avec le 4-hydroxybenzonitrile au sens d'une synthèse d'éther de Wilkinson ;
(f) conversion réductrice d'alkylesters d'acide 3-(4-cyano-phénoxy)benzoïque de formule (VII) 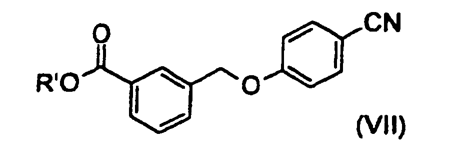 où R' représente C₁-C₄-alkyle, en un composé de formule (V) 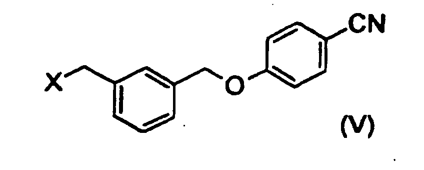 où X représente hydroxy ;
(g) éventuellement traitement du composé de formule (V) où X représente hydroxy avec un réactif d'halogénation ou un chlorure d'acide sulfonique ;
(h) réaction du composé de formule (V) où X représente hydroxy, le chlore, le brome, mésylate, triflate ou tosylate, avec un dérivé du phénol de formule (VI) 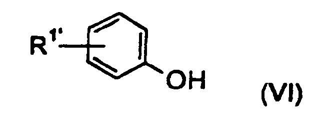 où R^{1'} présente les significations citées dans les revendications 1 à 4 ; éventuellement sous forme des phénolates de sodium ou de potassium correspondants, dans des conditions réactionnelles basiques dans un solvant organique polaire ;
(i) conversion du composé de formule (II) obtenu en le composé de formule (I) selon le procédé selon les revendications 1 à 3.

5. Produits intermédiaires de formule (IVA) et/ou leur forme tautomère 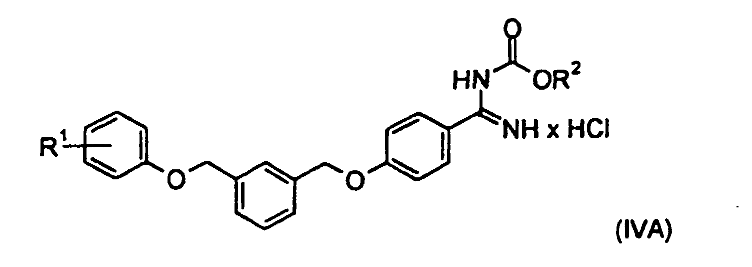 où les groupements R¹ et R² peuvent avoir les significations citées dans les revendications 1, 2 ou 3.

6. Produits intermédiaires de formule (V) 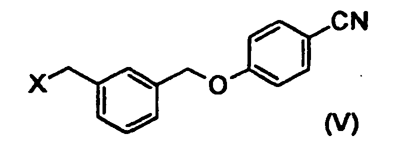 où X représente hydroxy, le chlore, le brome, mésylate, triflate ou tosylate.

7. Produits intermédiaires selon la revendication 6 où X représente hydroxy ou le chlore.

8. Produits intermédiaires de formule (VII) 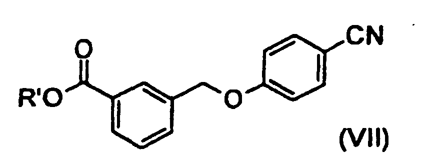 où R' représente C₁-C₄-alkyle.

9. Utilisation des produits intermédiaires de formule (IV) selon la revendication 5 pour la production d'un composé de formule (I) selon la revendication 1.

10. Utilisation des produits intermédiaires de formule (V) selon la revendication 6 ou 7 pour la production d'un composé de formule (I) selon la revendication 1.

11. Utilisation des produits intermédiaires de formule (VII) selon la revendication 8 pour la production d'un composé de formule (I) selon la revendication 1.
